(19) Europäisches Patentamt
European Patent Office
Office européen des brevets

(11) Veröffentlichungsnummer: **0 011 821**
A2

# (12) EUROPÄISCHE PATENTANMELDUNG

(21) Anmeldenummer: **79104639.4**

(22) Anmeldetag: **22.11.79**

(51) Int. Cl.[3]: **A 61 K 7/06**

(30) Priorität: **27.11.78 DE 2851283**

(43) Veröffentlichungstag der Anmeldung:
**11.06.80 Patentblatt 80/12**

(84) Benannte Vertragsstaaten:
**BE CH DE FR GB IT NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Möller, Hinrich, Dr.**
**Erlanger Strasse 45**
**D-4000 Düsseldorf 13(DE)**

(72) Erfinder: **Thimm, Hans-Joachim, Dr.**
**Kantstrasse 4-6**
**D-4010 Hilden(DE)**

## (54) Haarbehandlungsmittel mit Antischuppenwirkung.

(57) Haarbehandlungsmittel mit Antischuppenwirkung, mit einem Gehalt an substituierten Isochinolinen der Formel

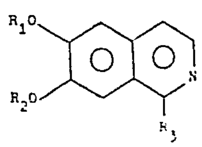

$R_1$, $R_2$ = $CH_3$, $C_2H_5$,

$R_3$ = Alkylgruppe mit 1 - 6 C-Atomen, gegebenenfalls durch 4-Cl-, 3,4-Dimethoxy-substituierter Phenylrest, Benzylrest (gegebenenfalls in $\alpha$-Stellung Alkylgruppe mit 1 - 6 C-Atomen, im Kern 4-Cl-, 3-, 4-Methoxy-, 3,4-Dimethoxy-, 3,4-Diethoxy- Substitution).

EP 0 011 821 A2

Croydon Printing Company Ltd.

4000 Düsseldorf, den 24.11.1978
Henkelstraße 67

HENKEL KGaA 0011821
ZR-FE/Patente
Z/Br

# P a t e n t a n m e l d u n g

## D 5856

## "Haarbehandlungsmittel mit Antischuppenwirkung"

Gegenstand der Erfindung sind Haarbehandlungsmittel mit Antischuppenwirkung, die als Wirkstoffe substituierte Isochinoline enthalten.

Haarbehandlungsmittel zur Beseitigung unerwünschter Schuppenbildung auf der Kopfhaut sind bereits in größerer Anzahl auf Basis der unterschiedlichsten Wirkstoffe und Zubereitungen bekannt. Einen bevorzugten Platz nehmen dabei die Haarbehandlungsmittel auf der Grundlage wäßriger Tensidlösungen wie Shampoos und Haarkurspülungen ein. Um als brauchbare Antischuppenmittel eingesetzt werden zu können, müssen derartige Produkte eine Reihe von Voraussetzungen erfüllen, die nachfolgend kurz aufgeführt werden.

Die Wirkstoffe müssen toxikologisch und dermatologisch unbedenklich sein und auch die damit hergestellten Haarbehandlungsmittel müssen sich durch eine gute Hautverträglichkeit auszeichnen. Die Antischuppenwirksamkeit der Kompositionen darf auch durch eine lange Lagerung, z.B. durch Reaktion mit anderen Bestandteilen des Mittels, nicht ungünstig beeinflußt werden. Die Wirkung des Mittels darf nicht zu schnell abklingen, sondern sie

/2

muß zumindest die Zeit zwischen zwei Behandlungen, seien es nun Haarwäschen oder andere Haarbehandlungen mit dem Antischuppenwirkstoff, überbrücken. Ferner soll der Antischuppenwirkstoff eine gute Verträglichkeit mit den üblicherweise verwendeten anderen Bestandteilen der Haarbehandlungsmittel aufweisen und im Hinblick auf Aussehen und Geruch ansprechend wirken.

Es wurde nun gefunden, daß diese Forderungen weitgehend erfüllt werden, wenn man Haarbehandlungsmittel mit Antischuppenwirkung verwendet, die Isochinolinderivate der allgemeinen Formel

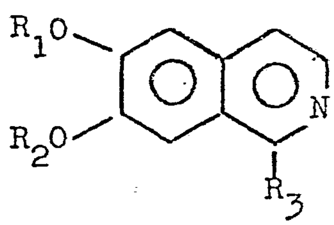

in der $R_1$ und $R_2$ für eine Methyl- oder Ethylgruppe und $R_3$ für eine niedere Alkylgruppe mit 1 - 6 C-Atomen, einen Phenylrest, der durch 4-Chlor oder 3,4-Dimethoxy substituiert sein kann oder einen Benzylrest, der in $\alpha$-Stellung durch eine niedere Alkylgruppe mit 1 - 6 C-Atomen und im Kern durch 4-Chlor, 3-, 4-Methoxy, 3,4-Dimethoxy, 3,4-Diethoxy substituiert sein kann, stehen, in einer Menge von 0,01 bis 5 Gewichtsprozent, vorzugsweise 0,5 bis 3 Gewichtsprozent, bezogen auf das gesamte Mittel, enthalten.

Anstelle der freien Isochinolinderivate können auch deren Säureadditionsprodukte mit physiologisch unbedenklichen Säuren, wie z.B. Chlorwasserstoff, Bromwasserstoff, Schwefelsäure, Phosphorsäure, Essigsäure, Propionsäure, Milchsäure, Zitronensäure, Nicotinsäure Verwendung finden.

/3

Besonders günstige schuppenhemmende Effekte weisen Haarbehandlungsmittel auf, die neben den erfindungsgemäß einzusetzenden Isochinolinderivaten Fettalkohol-polyalkylenoxid-Addukte, vorzugsweise solche von Fettalkoholen der Kettenlängen mit 12 - 18 Kohlenstoffatomen mit 5 - 40 Mol Ethylenoxid-Einheiten, enthalten.

Die in den erfindungsgemäßen Haarbehandlungsmitteln mit Antischuppenwirkung einzusetzenden Isochinolinderivate sind bekannte Verbindungen oder nach üblichen Verfahren der organischen Synthese herstellbar. Die Herstellung wird nach folgendem Schema vorgenommen:

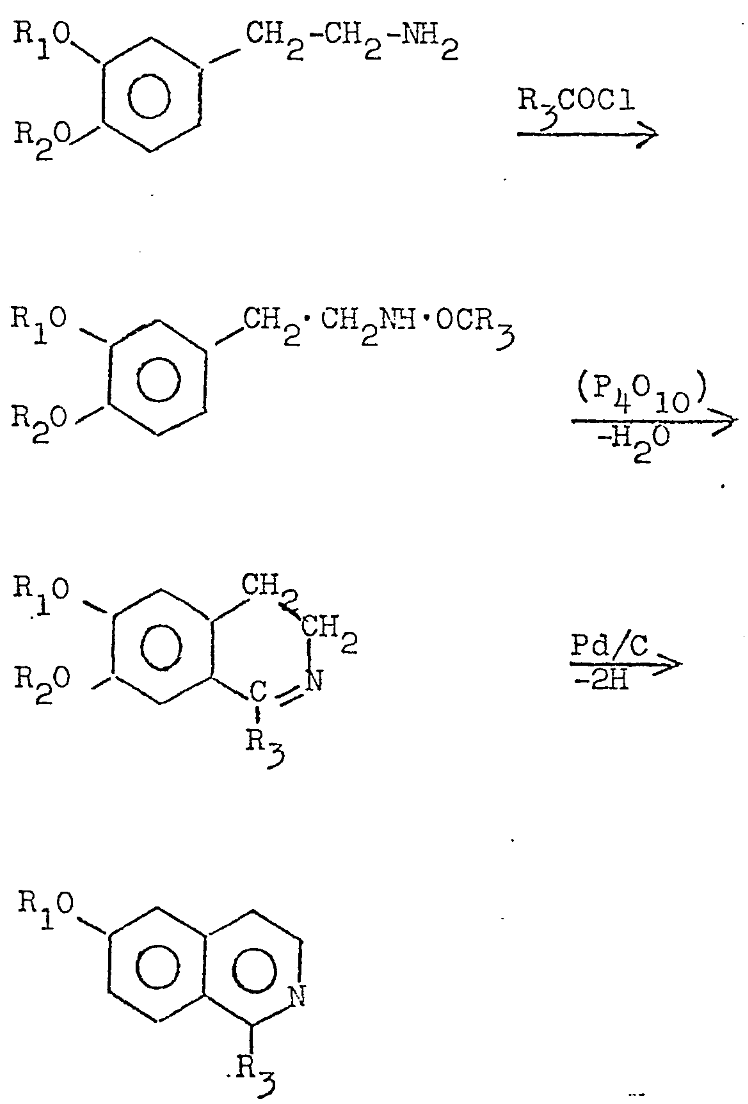

In diesen Formeln haben $R_1$, $R_2$ und $R_3$ die vorgenannte Bedeutung. Das als Ausgangsmaterial dienende 2-Arylethylamin ist z.B. durch Reduktion des entsprechenden Arylacetonitrils zugänglich. Die vorzunehmende Cyclokondensation mit $P_4O_{10}$ ist als Bischler-Napieralski-Reaktion bekannt. Die Herstellung von Papaverin kann in der Weise erfolgen, wie sie z.B. im Can. J. Chem. 33, 729 (1955) oder in den Ber. dtsch. Chem. Ges. 60, 392 (1927) beschrieben ist.

Als in den erfindungsgemäßen Haarbehandlungsmitteln mit Antischuppenwirkung einzusetzende Isochinolinderivate sind zum Beispiel 1-(3',4'-dimethoxybenzyl)-6,7-dimethoxyisochinolin (Papaverin), 1-(3',4'-dimethoxyphenyl)-6,7-dimethoxy-, 1-(4'-Chlorphenyl)-6,7-dimethoxy-, 1-Benzyl-6,7-dimethoxy-, 1-Ethyl-6,7-dimethoxy-, 1-(3',4'-diethoxybenzyl)-6,7-diethoxy-, 1-[α-(3',4'-dimethoxyphenyl)-ethyl]-6,7-dimethoxyisochinolin zu nennen.

Als erfindungsgemäße Haarbehandlungsmittel mit Antischuppenwirkung sind alle Arten der bisher für solche Zwecke verwendeten Mittel geeignet, wie z.B. Haarwasser, Frisiercremes, Haarwaschmittel, Haarfestiger, Haarkuren, Haarspülungen, Haarlotionen auf Basis von Wasser-in-Öl und Öl-in-Wasser-Emulsionen. Besondere Bedeutung kommt dabei den Haarwaschmitteln, Haarkuren und Haarspülungen zu. Derartige Produkte enthalten neben den 0,01 - 5 Gew.-%, vorzugsweise 0,5 - 3 Gew.-%, an substituierten Isochinolinen die darin üblichen Bestandteile in den gebräuchlichen Mengen.

Die erfindungsgemäßen Haarbehandlungsmittel können im einfachsten Fall aus einer wäßrigen oder wäßrig-alkoholischen Lösung beziehungsweise Dispersion des Wirkstoffes nebst Parfüm und Farbstoff bestehen und in dieser Form als Haarwasser oder Nachspülmittel einge-



BAD ORIGINAL

setzt werden. Die Mittel können in Anpassung an andere Verwendungszwecke, wie z.B. Haarshampoos, Haarkuren usw., weitere in derartigen Mitteln gebräuchliche Komponenten in üblichen Mengenverhältnissen enthalten.

Demgemäß können als tensidische Bestandteile Sulfattenside wie Fettalkoholsulfate, Fettalkoholethersulfate mit 3 bis 4 Ethylenoxideinheiten im Molekül, Alkylphenolethersulfate, Monoglyceridsulfate, ferner Fettsäure-Eiweißkondensationsprodukte, Fettsäuresarcoside und Fettsäuremethyltauride eingesetzt werden. Ferner kommen auch amphotere Tenside, wie z.B. die unter der Bezeichnung Miranole bekannten Imidazol-Abkömmlinge in Betracht. Die vorgenannten anionischen Tenside liegen insbesondere in Form ihrer Natrium- und Triethanolaminsalze, in Einzelfällen, wie z.B. Myristylalkoholethersulfat, auch in Form der Magnesiumsalze vor. Besonders günstige schuppenhemmende Effekte werden erzielt, wenn als tensidische Komponente Fettalkoholpolyalkylenoxid-Addukte, vorzugsweise solche aus Fettalkoholen mit 12 - 18 Kohlenstoffatomen mit 5 - 40 Ethylenoxid-Einheiten, in Kombination mit den Isochinolinderivaten und gegebenenfalls anderen oben genannten Tensiden eingesetzt werden.

Als emulgierend wirkende Bestandteile können in den erfindungsgemäßen Mitteln insbesondere Seifen der Stearin-, Laurin- und Ölsäure in Form ihrer Natrium-, Kalium- oder Alkanolaminsalze vorhanden sein, ferner die oben bereits genannten Sulfattenside, Polyol-Fettsäureester, z.B. Glycerinmonostearat, Propylenglykolmonostearat, Diethylenglykolmonostearat, zum Teil im Gemisch mit anionaktiven Emulgatoren, Fettalkoholgemische in Kombination mit anionaktiven Emulgatoren; nichtionische Emulgatoren wie Polyethylenoxidester, z.B. Polyoxyethylenstearat, -oleat usw.,

/6

Polyethylenoxidsorbitanester, einfache Sorbitanester wie Sorbitanmonolaurat, -oleat, -sesquioleat, Sterine, Polyethylenglykolester, wie z.B. die Mono- und Dilaurate, -oleate oder -stearate von Polyethylenglykol mit Molgewichten von 200 bis 600; kationaktive Emulgatoren wie Laurylammoniumchlorid, Cetylpyridiniumchlorid, Cetyltrimethylammoniumbromid, Diisobutylphenoxyethoxyethyldimethylbenzylammoniumchlorid, Alkyldimethylbenzylammoniumchlorid mit 10 - 14 C-Atomen im Alkylrest, N-(Stearylcolaminoformylmethyl)-pyridiniumchlorid o.ä.; Beispiele für ampholytische Emulgatoren sind Ethylencycloimido-1-lauryl-2-hydroxyethylennatriumalkoholat, Triethanolamin-β-alanin, N-Lauryl-aminopropionat, N-Lauryl-iminodipropionat, N-Lauryl-diethyltriaminoessigsäure.

Als gegebenenfalls zuzusetzende Verdickungsmittel können ebenfalls übliche Substanzen genommen werden, wie z.B. insbesondere Natriumalginatschleime, Fettsäurealkylolamide und zum Teil Tyloseschleime, ferner höhermolekulare Polyethylenglykolmono- oder diester von Fettsäuren, insbesondere der Stearin- und Laurinsäure. Als Verdickungsmittel kommen in einzelnen Fällen auch Elektrolyte wie Kochsalz oder Ammoniumchlorid in Kombination mit Alkylethersulfaten in Betracht.

Den erfindungsgemäßen Mitteln können in bestimmten Fällen ferner Überfettungsmittel zugesetzt werden, beispielsweise polyoxyethylierte Lanolinderivate, Lecithinderivate oder die bereits oben erwähnten Alkylolamide, denen eine gewisse auffettende Wirkung zukommt. Letztere können auch als Schaumstabilisatoren in Shampoos dienen.

Die erfindungsgemäßen Mittel können ferner sogenannte Gerüstbestandteile wie Paraffin, Fettstoffe, Lanolin und Wollfettalkohole, biogene Wirkstoffe wie Pflanzenextrakte,

/7

BAD ORIGINAL

Eiweißabbauprodukte und Vitaminkomplexe, Lösungsvermittler wie niedere Glykole, z.B. 1,3-Propandiol, 1,3-Butylenglykol, Diethylenglykol, Filmbildner wie Polyvinylpyrrolidon, Vinylpyrrolidon-Vinylacetat-Copolymerisate, Polymere der Acrylsäurereihe, Dimethylhydantoin-Formaldehydharze, Polymere der 2-Alkyl-2-oxazolin-Reihe enthalten.

Die nachfolgenden Beispiele sollen den Gegenstand vorliegender Erfindung näher erläutern, ohne ihn jedoch hierauf zu beschränken.

# Beispiele

Für die Prüfung auf Antischuppenwirkung wurde die Verbindung 1-(3',4'-dimethoxybenzyl)-6,7-dimethoxy-isochinolin (Papaverin) eingesetzt.

Die Prüfung auf Antischuppenwirkung wurde in folgender Weise durchgeführt:

Die Prüfsubstanz wurde in 5 %iger Konzentration zusammen mit 5 % Ölsäure in 70 %igem Ethanol gelöst und in alternierender Reihenfolge und in rotierendem Schema auf den Rücken junger, geschorener weiblicher Meerschweinchen im Gewicht von 600 ± 50 g aufgetragen. Die Einwirkungszeit der Substanz wurde dabei auf 60 Minuten begrenzt, da diese Zeit ausreichte, um eine Wirkung zu erzielen. Anschließend wurden die Tiere mit warmem Wasser 30 Sekunden lang abgeduscht. Die Reihenfolge der Tiere bei der Applikation wechselte dabei täglich, um Differenzen in der Einwirkungszeit zwischen dem ersten und letzten Tier der Gruppe auf ein Minimum zu begrenzen. Diese Behandlung geschah an vier aufeinanderfolgenden Tagen, darauf folgte eine Ruhezeit (Entwicklungszeit von drei Tagen). Am siebenten Tag wurde ausgewertet, und zwar a) kutimetrisch auf Hautfaltendicke instrumentell mit dem Cutimeter nach Götze und b) visuell auf Schuppenbildung bzw. Hemmung nach einer 4-Punkte-Skala folgender Definition:

0 = keine Schuppen
1 = leichte Schuppen
2 = kräftige Schuppen
3 = starke Schuppen mit Rötung
4 = Krusten mit Aufrichten der Haare.

/9

Die Versuchsgruppe bestand aus 6 Tieren. Das Kollektiv wurde mathematisch auf Homogenität geprüft und nach Eliminierung signifikant abweichender Tiere wurden Mittelwert und Streuung berechnet. Diese wurden in Beziehung gesetzt zu den Werten der nur mit 5 % Ölsäure in 70 %igem Ethanol behandelten Haut und daraus die Signifikanz der Hemmwirkung bestimmt. Das Ergebnis der Prüfung ist der nachstehenden Tabelle 1 zu entnehmen.

Tabelle 1

| Hautdickenmessung | | Hautschuppenmessung | |
|---|---|---|---|
| Hemmung % | (sign.)x) | Hemmung % | (sign.)x) |
| 17 | + | 100 | + |

x) signifikant mit 95 %iger Wahrscheinlichkeit. Durch das Ergebnis der Versuche wurde die gute Antischuppenwirkung der erfindungsgemäß zu verwendenden Derivate vollauf bestätigt.

Nachstehend werden einige Beispiele für Haarbehandlungsmittel mit Antischuppenwirkung aufgeführt.

Beispiel 1 Alkoholisches Antischuppenhaarwasser

| | | |
|---|---|---|
| 1-Benzyl-6,7-dimethoxy-isochinolin | 3,0 | Gew.-Teile |
| Parfüm | 0,5 | " |
| Ethylalkohol 96 %ig | 67,0 | " |
| Wasser | 29,5 | " |

Beispiel 2 Haarfestlegemittel mit Antischuppenwirkung

| | | |
|---|---|---|
| Copolymerisat aus 60 Gew.-% Vinylpyrrolidon, 40 Gew.-% Vinylacetat | 2,0 | Gew.-Teile |
| Sorbitanmonolaurat | 0,4 | " |
| Pflanzenextrakt | 0,5 | " |
| Parfüm | 0,6 | " |
| 1-Ethyl-6,7-dimethoxy-isochinolin | 2,5 | " |
| Isopropanol | 34,0 | " |
| Wasser | 60,0 | " |

Beispiel 3 Antischuppenshampoo

| | | |
|---|---|---|
| Natriumlaurylethersulfat 27 % Aktivsubstanz | 50,0 | Gew.-Teile |
| Kokosfettsäurediethanolamid | 2,0 | " |
| Polydiol 400 | 0,5 | " |
| Parfümöl | 0,3 | " |
| Kosmetikfarbstoff blau 0,1 %ig | 3,0 | " |
| Natriumalginat | 2,0 | " |
| Papaverin | 1,0 | " |
| Wasser | 41,2 | " |

Beispiel 4 Antischuppenshampoo

| | | |
|---|---|---|
| Natriumlaurylethersulfat 27 % Waschaktivsubstanz | 63,0 | Gew.-Teile |
| Kokosfettsäurediethanolamid | 2,0 | " |
| Ethylenglykolstearinsäureester | 1,0 | " |
| Natriumchlorid | 2,0 | " |
| Proteinhydrolysat | 0,5 | " |
| Parfümöl | 0,5 | " |
| 1,-(3',4'-dimethoxy-phenyl)-6,7-dimethoxy-isochinolin | 2,0 | " |
| Wasser | 29,0 | " |

/11

Beispiel 5 Haarspülmittel mit Antischuppenwirkung

| | | |
|---|---|---|
| Wollfett | 1,0 | Gew.-Teile |
| Paraffinöl | 1,0 | " |
| Glyceridgemisch | 2,0 | " |
| Isopropylmyristat | 2,0 | " |
| Cetyl-stearylalkohol | 0,4 | " |
| Glycerin | 10,0 | " |
| Cetylpyridiniumchlorid | 0,5 | " |
| Natriumalginat | 0,2 | " |
| Parfümöl | 0,5 | " |
| 1-(4'-Chlor-phenyl)-6,7-dimethoxy-isochinolin | 2,0 | " |
| Wasser | 80,4 | " |

Beispiel 6 Haarkurmittel mit Antischuppenwirkung

| | | |
|---|---|---|
| Wollfett | 1,0 | Gew.-Teile |
| Paraffinöl | 1,0 | " |
| Cetyl-stearylalkohol | 1,0 | " |
| Glyceridgemisch | 7,0 | " |
| Isopropylmyristat | 1,0 | " |
| Glycerin | 10,0 | " |
| Sorbitanmonolaurat | 1,0 | " |
| Pflanzenextrakt | 1,0 | " |
| Cetylpyridiniumchlorid | 0,5 | " |
| Natriumalginat | 0,2 | " |
| Parfümöl | 0,3 | " |
| 1-(3',4'-diethoxybenzyl)-6,7-diethoxy-isochinolin | 3,0 | " |
| Wasser | 73,0 | " |

<u>Beispiel 7</u> Antischuppenshampoo

| | | |
|---|---|---|
| Cetylstearylalkohol mit ca. 40 Mol EO | 17,0 | Gew.-Teile |
| Kokosfettsäurediethanolamid | 2,0 | " |
| Ethylenglykolstearinsäureester | 1,0 | " |
| Cetylpyridiniumchlorid | 0,5 | " |
| Proteinhydrolysat | 0,5 | " |
| Papaverin | 0,5 | " |
| Parfümöl | 0,5 | " |
| Wasser | 78,0 | " |

<u>Beispiel 8</u> Antischuppenshampoo

| | | |
|---|---|---|
| Natriumlaurylethersulfat 27 % Waschaktivsubstanz | 36,0 | Gew.-Teile |
| Cetylstearylalkohol mit ca. 30 Mol EO | 7,5 | " |
| Kokosfettsäurediethanolamid | 2,0 | " |
| Ethylenglykolstearinsäureester | 1,0 | " |
| Natriumchlorid | 1,5 | " |
| Proteinhydrolysat | 0,5 | " |
| Parfümöl | 0,5 | " |
| 1-[α-(3',4'-dimethoxyphenyl)-ethyl]-6,7-dimethoxy-isochinolin | 1,0 | " |
| Wasser | 50,0 | " |

"Haarbehandlungsmittel mit Antischuppenwirkung"

Patentansprüche:

1. Haarbehandlungsmittel mit Antischuppenwirkung, dadurch gekennzeichnet, daß es in einem geeigneten Trägermaterial Isochinolinderivate der allgemeinen Formel

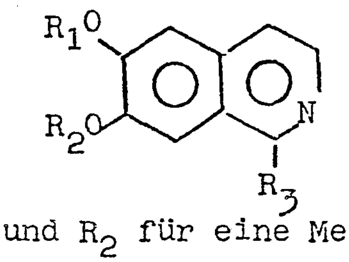

in der $R_1$ und $R_2$ für eine Methyl- oder Ethylgruppe und $R_3$ für eine niedere Alkylgruppe mit 1 - 6 C-Atomen, einen Phenylrest, der durch 4-Chlor oder 3,4-Dimethoxysubstituiert sein kann oder einen Benzylrest, der in α-Stellung durch eine niedere Alkylgruppe mit 1 - 6 C-Atomen und im Kern durch 4-Chlor, 3-, 4-Methoxy-, 3,4-Dimethoxy, 3,4-Diethoxy substituiert sein kann, stehen, in einer Menge von 0,01 bis 5 Gewichtsprozent, vorzugsweise 0,5 bis 3 Gewichtsprozent, bezogen auf das gesamte Mittel, enthält.

2. Haarbehandlungsmittel mit Antischuppenwirkung nach Anspruch 1, dadurch gekennzeichnet, daß es neben den Isochinolinderivaten als tensidische Komponente Fettalkohol-polyalkylenoxid-Addukte, vorzugsweise solche von Fettalkoholen der Kettenlängen mit 12 - 18 Kohlenstoffatomen mit 5 - 40 Mol Ethylenoxideinheiten, enthält.